Europäisches Patentamt

European Patent Office

Office européen des brevets

(11) Numéro de publication: **0 379 433**

**A1**

(12) # DEMANDE DE BREVET EUROPEEN

(21) Numéro de dépôt: **90400137.7**

(22) Date de dépôt: **18.01.90**

(51) Int. Cl.⁵: **B01J 23/88, C07C 5/48, C07C 11/06**

(30) Priorité: **18.01.89 FR 8900552**

(43) Date de publication de la demande:
**25.07.90 Bulletin 90/30**

(84) Etats contractants désignés:
**AT BE CH DE DK ES FR GB GR IT LI LU NL SE**

(71) Demandeur: **NORSOLOR, Société Anonyme dite:**
**18 place des Reflets Tour Aurore**
**F-92080 Paris la Défense 2(FR)**

(72) Inventeur: **Mazzocchia, Carlo**
**Via Teodosio 33**
**Milano(IT)**
Inventeur: **Tempesti, Ezio**
**V. 1e Abruzzi 1**
**Milano(IT)**
Inventeur: **Aboumrad, Chafic**
**Via Val di non 4**
**Milano(IT)**

(74) Mandataire: **Chaillot, Geneviève**
**Cabinet CHAILLOT 21, avenue Louise de Bettignies**
**F-92700 Colombes(FR)**

(54) **Catalyseur et procédé de déshydrogénation oxydante du propane.**

(57) Catalyseur de formule

$Ni_a Mo O_x$     (I)

dans laquelle :
- a est un nombre de 0,6 à 1,3, et
- x est un nombre déterminé par les exigences de valence du nickel et du molybdène.
   La fabrication de ce catalyseur comprend la préparation d'un précurseur solvaté et en outre la décomposition thermique dudit précurseur solvaté pendant une durée de 1 à 4 heures et à une température $T_1$ de 520 à 600°C.
   Application à la déshydrogénation oxydante du propane à une température de 400° à 700°C.

EP 0 379 433 A1

## CATALYSEUR ET PROCEDE DE DESHYDROGENATION OXYDANTE DU PROPANE

La présente invention se rapporte à un procédé catalytique de déshydrogénation du propane pour former la mono-oléfine correspondante.

La littérature a déja fait état de nombreux catalyseurs utiles pour la déshydrogénation oxydante des hydrocarbures paraffiniques. On peut ainsi citer les oxydes mixtes de nickel et d'étain (brevet US-A-3 745 294 et US-A-3 801 671), les oxydes mixtes de chrome et de magnésium (brevet US-A- 3 801 672), ainsi que des oxydes complexes associant un métal du groupe VIII à un métal du groupe VI B de la Classification Périodique et, le cas échéant, à un métal du groupe IV B ou du groupe I A (brevets US-A-3 784 485 et US-A-4 476 339).

On connaît par le brevet allemand n° 1 800 063 un procédé pour l'obtention d'hydrocarbures mono- ou dioléofiniques par déshydrogénation oxydante catalytique du butane à une température de 400° à 700° C, caractérisé en ce que, comme catalyseur, on utilise un mélange d'oxydes comprenant un oxyde de molybdène ou tungstène et au moins un oxyde d'un métal choisi parmi chrome, manganèse, fer, nickel et cadmium. Ce document recommande plus particulièrement l'emploi de mélanges d'oxydes de molybdène et nickel dans un rapport du molybdène au nickel compris entre 4 et 0,04. L'exemple 1 illustre l'oxydéshydrogénation de n-butane au moyen d'un tel catalyseur, obtenu à partir de solutions aqueuses de molybdate d'ammonium et de nitrate de nickel, ce qui conduit à 590° C à un rendement de 4,5% en n-butène et de 21% en butadiène. L'exemple 2 montre qu'en effectuant la même réaction à 597° C avec un rapport atomique du nickel au molybdène égal à 0,68, on obtient des rendements en butadiène et butènes inférieurs à ceux obtenus dans l'exemple 1 avec un rapport atomique $\frac{Ni}{Mo}$ égal à 2.

Par ailleurs, le brevet US-A-4 131 631 décrit un procédé de déshydrogénation d'hydrocarbures paraffiniques ayant de 3 à 6 atomes de carbone pour former les monooléfines correspondantes, par mise en contact de l'hydrocarbure avec de l'oxygène moléculaire, à une température de 400° à 700° C et sous une pression de 1 à 3 atmosphères, sur un catalyseur de formule :

$A_a Co_b Mo_c O_x$

dans laquelle :
- A est un élément choisi parmi le phosphore et les métaux des groupes IA, IIA, VI et VIII de la Classification Périodique,
- a est un nombre de 0 à 3,
- b est un nombre de 0,1 à 2,
- c est un nombre de 0,1 à 6, et
- x est un nombre déterminé par les exigences de valence des éléments A, Co et Mo.

Plus particulièrement, l'exemple 8 de ce brevet décrit un catalyseur de $Co_{0,5} Ni_{0,5} Mo O_x$ supporté sur silice, soumis à calcination à 593° C pendant 24 heures. Ce catalyseur utilisé pour l'oxydéshydrogénation du propane à 538° C permet d'atteindre une conversion de 13,3% par passe avec une sélectivité de 63,9% en propylène, soit un rendement en propylène de 8,5%. Ce type de catalyseur pose toutefois d'insolubles problèmes de reproductibilité, dus à la présence de la silice en tant que support, et présente l'inconvénient d'un rendement insuffisant pour l'oxydéshydrogénation du propane. La durée de son processus de fabrication constitue par ailleurs un inconvénient économique évident.

L'objectif de la présente invention consiste donc en la mise au point d'un catalyseur reproductible, permettant, contrairement aux enseignements de l'art antérieur rappelé ci-dessus, d'oxydéshydrogéner le propane avec un rendement élevé.

Un premier objet de la présente invention consiste en un catalyseur de déshydrogénation oxydante du propane, de formule :

$Ni_a Mo O_x$ (I)

dans laquelle :
- a est un nombre de 0,6 à 1,3 environ, et
- x est un nombre déterminé par les exigences de valence du nickel et du molybdène.

Un catalyseur préféré selon l'invention est $NiMoO_4$

Le catalyseur selon l'invention peut comprendre en mélange un diluant solide et inerte notamment lorsqu'il est souhaitable d'éviter que des points chauds ne se créent en certaines zones de la surface catalytique. Parmi ces diluants, qui peuvent être utilisés à raison de jusqu'à environ 300% en poids du catalyseur, on peut citer notamment le carbure de silicium (carborundum).

Le catalyseur selon l'invention est de préférence un catalyseur massique, c'est-à-dire un catalyseur non supporté, de façon à résoudre les problèmes de reproductibilité mentionnés plus haut. Il possède une surface spécifique généralement comprise entre environ 20 et 50 m²/g et une granulométrie moyenne généralement comprise entre 50 et 2500 mesh environ (0,1 à 5 mm environ).

Dans la formule générale, mentionnée plus haut, du catalyseur selon l'invention, il est généralement préférable que le rapport x/a du nombre d'atomes d'oxygène au nombre d'atomes de nickel soit compris entre 3,5 et 6 environ.

Un second objet de la présente invention consiste en un procédé de fabrication du catalyseur de formule indiquée ci-dessus, comprenant la préparation d'un précurseur solvaté de formule générale :

a NiO, MoO$_3$, nH$_2$O, mNH$_3$

dans laquelle a possède la même signification que ci-dessus, par réaction du molybdate d'ammonium et du nitrate de nickel, et caractérisé en ce qu'il comprend en outre la décomposition thermique dudit précurseur solvaté pendant une durée de 1 à 4 heures environ et à une température T$_1$ de 520° à 600° C environ. Cette étape de décomposition thermique dans les conditions spécifiées s'est révélée nécessaire à l'obtention de bons résultats catalytiques dans l'oxydéshydrogénation du propane alors que l'art antérieur procède généralement à un simple séchage dans l'air. Facultativement, ce procédé de fabrication peut comprendre une étape finale d'activation thermique du précurseur décomposé, pendant une durée de 5 à 30 minutes environ et à une température T$_2$ supérieure à T$_1$, de préférence, comprise entre 600° et 750° C environ. L'étape initiale de formation du précurseur solvaté est bien connue et peut être effectuée notamment en mélangeant sous agitation des solutions de molybdate d'ammonium et de nitrate de nickel, à une température généralement comprise entre 65° et 90° C environ, et à un pH d'environ 5,6. Comme il est bien connu de l'homme de l'art, de petites variations des paramètres de cette préparation (par exemple le pH, la température de précipitation, la concentration, la température de filtration, la température et la durée du séchage) permettent la formation de précurseurs solvatés ayant des valeurs a, m et n différentes. Ainsi, on a préparé des précurseurs solvatés de la formule générale ci-dessus, dans laquelle n≤1 et 0≤m≤1, en effectuant un séchage pendant 5 heures à 120° C. C'est notamment le cas dans les exemples ci-après. Comme il va de soi pour des techniques de décomposition thermique (température T$_1$) et d'activation thermique (température T$_2$), la durée de ces étapes est d'autant plus courte que la température choisie pour le traitement thermique (T$_1$ et T$_2$) est plus élevée. Ainsi dans les cas extrêmes les plus longs, la durée des traitements thermiques combinés selon l'invention est-elle cinq fois plus courte que celle décrite par le brevet US-A-4 131 631.

Un troisième objet de la présente invention consiste en un procédé de déshydrogénation du propane comprenant la mise en contact du propane avec de l'oxygène moléculaire, à une température d'environ 400° à 700° C, et, de préférence, 550° à 650° C, et sous pression ne dépassant pas 0,4 MPa environ (4 atmosphères), en présence d'un catalyseur possédant la formule générale (I) décrite ci-dessus ou obtenu par le procédé de fabrication décrit ci-dessus. Le temps de contact apparent utilisé dans ce procédé peut être choisi dans une gamme allant de 0,01 à 30 secondes, et de préférence 0,1 à 5 secondes. Le procédé selon l'invention doit être réalisé en présence d'oxygène moléculaire ou d'un gaz contenant de l'oxygène moléculaire tel que l'air. Le rapport molaire entre le propane et l'oxygène dans le procédé selon l'invention est généralement compris entre 0,1 et 30, et de préférence entre 0,5 et 10. Le mélange réactionnel peut en outre être dilué par au moins un gaz inerte tel que l'azote, le dioxyde de carbone, la vapeur d'eau, etc. La concentration en propane dans le mélange réactionnel peut sans inconvénient atteindre jusqu'à 25% en moles.

Le procédé de déshydrogénation oxydante selon l'invention peut être mis en oeuvre soit dans un réacteur classique, tel qu'un réacteur à lit fixe ou un réacteur à lit fluidisé.

Les exemples spécifiques ci-après permettront une meilleure compréhension de l'invention. Toutefois, ils ne sont donnés qu'à titre indicatif et non limitatif.

EXEMPLES 1 et 2

Préparation du catalyseur (A)

On mélange sous agitation deux solutions équimoléculaires de molybdate d'ammonium et de nitrate de nickel à pH 5,6 et à une température de 85° C, de manière à obtenir un précurseur précipité de formule générale :

NiO, MoO$_3$, n H$_2$O, m NH$_3$ .

Ce précurseur est ensuite décomposé thermiquement pendant deux heures à 550° C, de manière à obtenir un catalyseur (A) de formule NiMoO$_4$ et possédant une surface spécifique de 40 m$^2$/g.

Pour la déshydrogénation oxydante du propane, on utilise un réacteur horizontal tubulaire en quartz, de

3

volume total 30 cm³, relié à quatre débitmètres massiques alimentés successivement par l'azote, l'oxygène, le propane et l'air. 0,5 g de catalyseur (A) mélangé à 10 g de poudre de carborundum (SIC) est bloqué dans la partie centrale du réacteur entre deux tampons de carborundum de forte granulation et deux tampons de laine de quartz aux extrémités. Au centre du catalyseur se trouve installée une gaine en quartz permettant d'introduire un thermocouple et de mesurer la températrure effective du lit catalytique. Le chauffage est assuré par des résistances électriques dans la chambre du réacteur où le conducteur est l'air chaud en agitation continue. Le rapport molaire propane/oxygène est égal à 0,9.

On fait passer les composants du mélange réactionnel sur le lit catalytique à la vitesse de 15 litres normaux par heure et à la température T (exprimée en degrés Celsius) indiquée au tableau ci-après. Les produits de la réaction sont refroidis à 100° C avant d'être analysés par chromatographie en phase gazeuse pour déterminer leur ccmposition et notamment la concentration en propylène.

La conversion molaire indiquée dans le tableau ci-après, et exprimée en %, représente le propane total converti en propylène et en d'autres produits (conversion par passe) ; la sélectivité en propylène indiquée dans le tableau ci-après représente le pourcentage de propylène obtenu par rapport au propane converti ; enfin le rendement (conversion molaire en propylène par passe) indiqué dans le tableau ci-après représente le pourcentage de propane converti en propylène.

## EXEMPLES 3 et 4

### Préparation du catalyseur (B)

Après avoir été mélangé à la poudre de carborundum et installé dans le réacteur, le catalyseur (A) est soumis à une activation thermique. Pour cela, le réacteur est porté, en présence d'oxygène, à 700° C en l'espace de 25 minutes, puis laissé pendant 5 minutes à 700° C avant refroidissement jusqu'à la température de réaction. On obtient ainsi un catalyseur activé (B) qui est utilisé dans les mêmes conditions qu'aux exemples précédents. Le tableau ci-après indique les résultats obtenus en fonction de la température réactionnelle.

## EXEMPLE 5

### Préparation du catalyseur (C)

On reproduit la procédure de préparation du catalyseur (A), à l'exception suivante près : les solutions de molybdate d'ammonium et de nitrate de nickel sont mélangées à la température de 70° C à pH 5,6, puis le précipité formé est maintenu à séjourner jusqu'à avoir une quantité d'anhydride molybdique en excès telle que, après décomposition thermique pendant deux heures à 550° C, le catalyseur (C) obtenu ait pour formule $Ni\ Mo_{1,5}O_{5,5}$. Ce catalyseur possède une surface spécifique de 27 m²/g. Il est utilisé dans les mêmes conditions qu'aux exemples 2 et 4. Les résultats obtenus figurent au tableau ci-après.

## EXEMPLE 6 (Comparatif)

### Préparation du catalyseur (D)

On reproduit la procédure de préparation du catalyseur (A) à l'exception suivante près : les solutions de molybdate d'ammonium et de nitrate de nickel sont mélangées à la température de 85° C à pH 6. A cause de la coprécipitation d'un composé avec un excès de nickel, on obtient un précurseur de couleur verte qui, après décomposition thermique pendant deux heures à 550° C, fournit un catalyseur (D) de formule $Ni_{1,5}\ MoO_{4,5}$ et de surface spécifique 30 m²/g. Ce catalyseur est utilisé dans les mêmes conditions qu'aux exemples précédents. Les résultats obtenus figurent au tableau ci-après.

Tableau

| Exemple | T | Conversion | Sélectivité | Rendement |
|---------|-----|------------|-------------|-----------|
| 1 | 560 | 23,3 | 50,6 | 11,8 |
| 2 | 600 | 37,1 | 33,8 | 12,5 |
| 3 | 560 | 16,8 | 80,3 | 13,5 |
| 4 | 600 | 29,0 | 62,5 | 18,1 |
| 5 | 600 | 37,2 | 28,6 | 10,6 |
| 6 | 600 | 34,0 | 18,5 | 6,3 |

## Revendications

1 - Catalyseur de déshydrogénation oxydante du propane, de formule :

$Ni_a Mo O_x$     (I)

dans laquelle :

- a est un nombre de 0,6 à 1,3, et
- x est un nombre déterminé par les exigences de valence du nickel et du molybdène.

2 - Catalyseur selon la revendication 1, caractérisé en ce que a = 1 et x = 4.

3 - Procédé de fabrication du catalyseur selon la revendication 1, comprenant la préparation d'un précurseur solvaté de formule générale :

$a NiO, MoO_3, n H_2O, m NH_3$

par réaction du molybdate d'ammonium et du nitrate de nickel, caractérisé en ce qu'il comprend en outre la décomposition thermique dudit précurseur solvaté pendant une durée de 1 à 4 heures et à une température $T_1$ de 520° à 600° C.

4 - Procédé selon la revendication 3, caractérisé en ce qu'il comprend une étape finale d'activation thermique du précurseur décomposé pendant une durée de 5 à 30 minutes et à une température $T_2$ supérieure à $T_1$, comprise entre 600° et 750° C.

5 - Procédé de déshydrogénation du propane en propène, comprenant la mise en contact du propane avec de l'oxygène moléculaire à une température de 400° à 700° C et sous pression ne dépassant pas 0,4 MPa et en présence d'un catalyseur, caractérisé en ce que le catalyseur est conforme à la revendication 1.

6 - Procédé selon la revendication 5, caractérisé en ce que le rapport molaire entre le propane et l'oxygène est compris entre 0,1 et 30.

7 - Procédé selon l'une des revendications 5 et 6, caractérisé en ce que le mélange réactionnel est dilué par au moins un gaz inerte, la concentration en propane dans ledit mélange atteignant jusqu'à 25% en moles.

8 - Catalyseur selon l'une des revendications 1 et 2, caractérisé en ce qu'il possède une surface spécifique comprise entre 20 et 50 m²/g.

9 - Catalyseur selon l'une des revendications 1 et 2, caractérisé en ce qu'il comprend en mélange un diluant solide inerte.

Office européen
des brevets

**RAPPORT DE RECHERCHE EUROPEENNE**

Numero de la demande

EP 90 40 0137

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | DE-A-1 800 063 (NAUCNO-ISSL. INSTITUT) * Pages 2-3; exemple 1; revendications 1,2 * | 1-5 | B 01 J 23/88<br>C 07 C 5/48<br>C 07 C 11/06 |
| A | US-A-4 250 346 (YOUNG) | | |
| A | GB-A-2 068 250 (NIKKI) | | |
| A | US-A-3 303 236 (J.L. CROCE) | | |
| D,A | US-A-4 131 631 (HARDMAN) | | |
| D,A | US-A-3 801 671 (R.M. MARSHECK) | | |

**DOMAINES TECHNIQUES RECHERCHES (Int. Cl.5)**

B 01 J
C 07 C

**Le présent rapport a été établi pour toutes les revendications**

| Lieu de la recherche | Date d'achèvement de la recherche | Examinateur |
|---|---|---|
| LA HAYE | 20-03-1990 | LO CONTE C. |